# EUROPÄISCHE PATENTSCHRIFT

(11) **EP 0 805 145 B1**
(45) Veröffentlichungstag und Bekanntmachung des Hinweises auf die Patenterteilung: **31.10.2001**
(21) Anmeldenummer: 97106550.3
(22) Anmeldetag: 21.04.1997
(51) Int. Cl.: C07C 227/32, C07C 229/48, C07C 69/74, C07C 271/24

(54) **Effizientes und hochenantioselektives Verfahren zur Herstellung von enantiomerenreinen Cyclopentan-beta-Aminosäuren**
Efficient and highly enantioselective process for the preparation of enantiomerically pure cylopentane-beta-amino acids
Procédé de préparation efficace et hautement sélectif d'acide bèta amino cyclopentane carboxylique énantiomériquement pur

(30) Priorität: 03.05.1996 DE 19617772
(43) Veröffentlichungstag der Anmeldung: 05.11.1997
(73) Patentinhaber: BAYER AG, 51368 Leverkusen (DE)
(72) Erfinder: Mittendorf, Joachim, Dr., 42113 Wuppertal (DE)

(56) Entgegenhaltungen:
- WO-A-95/19337
- TETRAHEDRON: ASYMMETRY (1992), 3(2), 199-200, XP002036042 CHENEVERT, ROBERT ET AL: "Enantioselective synthesis of (+)- and (-)-cis-3- aminocyclopentanecarboxylic acids by enzymatic asymmetrization"
- TETRAHEDRON LETT. (1987), 28(47), 5841-4, XP002036043 FURUTA, KYOJI ET AL: "Asymmetric Diels -Alder reaction. A facile route to chiral alkyl hydrogen cyclohexene-1,2-dicarboxylates"
- JOURNAL OF THE AMERICAN CHEMICAL SOCIETY, Bd. 94, Nr. 17, 1972, DC US, Seiten 6203-05, XP002036044 TAKAYUKI SHIOIRI ET AL.: "Diphenylphosphoryl Azide. A new convenient reagent for a modified curtiun reaction and for the peptide synthesis"

## Beschreibung

Die vorliegende Erfindung betrifft ein effizientes und hochenantioselektives Verfahren zur Herstellung von enantiomerenreinen Cyclopentan- β-aminosäuren.

Aus den Publikationen EP 571 800, JP 021 7747 53 A2 und J. Antibiot. (1991), 44 (5), 546-9 sind Cyclopentan-β-aminosäuren bekannt. In der Publikation WO 95/19337 wird ein hochenantioselektives Verfahren zur Herstellung von enantiomerenreinen Cyclopentan- und -penten-β-aminosäuren beschrieben. Diese werden ausgehend von den entsprechenden meso-Dicarbonsäureanhydriden in sechs Synthesestufen mit Gesamtausbeuten von 28-40% d.Th. mit einem Enantiomerenüberschuß von ≥ 98% erhalten.

Gegenstand der Erfindung ist ein effizientes und hochenantioselektives Verfahren zur Herstellung von enantiomerenreinen Cyclopentan-β-aminosäuren der allgemeinen Formel (I) 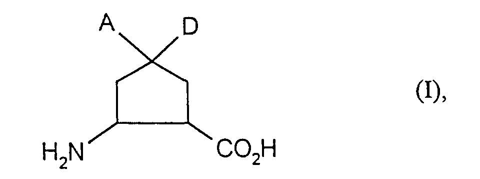 in welcher
- A und D: gleich oder verschieden sind und für Wasserstoff, Halogen, Hydroxy, oder für geradkettiges oder verzweigtes Alkyl mit bis zu 8 Kohlenstoffatomen, das gegebenenfalls 1- bis 2-fach gleich oder verschieden durch Halogen, Hydroxy, Phenyl, Benzyloxy, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR⁷R⁸ substituiert ist,
worin R⁷ und R⁸ gleich oder verschieden sind und Wasserstoff, Phenyl oder geradkettiges oder verzweigtes Alkyl mit bis zu 6 Kohlenstoffatomen bedeuten, stehen
oder
- A und D: gemeinsam für einen Rest der Formel 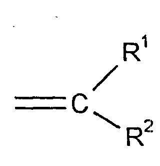 stehen,
worin
R¹ und R² gleich oder verschieden sind und Wasserstoff, Halogen oder geradkettiges oder verzweigtes Alkyl, Alkoxy oder Oxyacyl mit bis zu 8 Kohlenstoffatomen, Benzyl oder Phenyl bedeuten,
das dadurch gekennzeichnet ist, daß man meso-Dicarbonsäureanhydride der allgemeinen Formel (II) 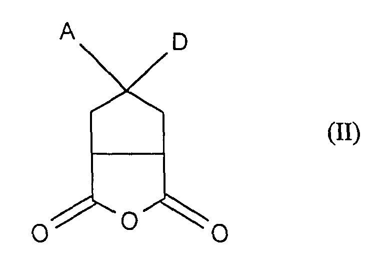 in welcher
- A und D: die oben angegebenen Bedeutungen haben,
durch eine asymmetrische Alkoholyse mit Allylalkoholen der allgemeinen Formel (III) 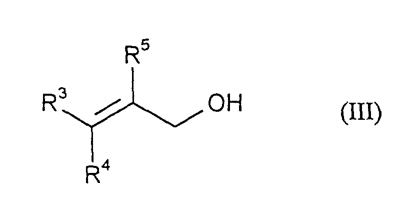 in welcher
- R³, R⁴ und R⁵: gleich oder verschieden sind und für Wasserstoff oder für geradkettiges oder verzweigtes Alkyl mit bis zu 5 Kohlenstoffatomen oder für Phenyl stehen, das gegebenenfalls bis zu 3-fach gleich oder verschieden durch Halogen, Cyano, Trifluormethoxy, Nitro, Trifluormethyl oder durch geradkettiges oder verzweigtes Alkyl oder Alkoxy mit jeweils bis zu 6 Kohlenstoffatomen substituiert ist,
oder
- R³: für einen 5- bis 7-gliedrigen aromatischen Heterocyclus mit bis zu 3 Heteroatomen aus der Reihe S, N und/oder O steht,
und in Anwesenheit equimolarer Mengen einer in enantiomerenreiner Form vorliegenden chiralen Aminbase, in inerten Lösemitteln, zunächst über die intermediäre, enantiomerenreine Salzstufe der allgemeinen Formel (IV) 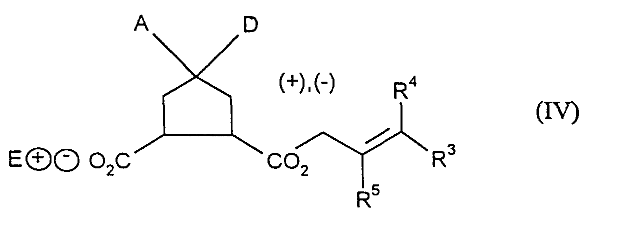 in welcher
- A, D, R³, R⁴ und R⁵: die oben angegebenen Bedeutungen haben
und
- E: für die chirale Aminbase steht,
in die enantiomerenreinen Verbindungen der allgemeinen Formel (IVa) 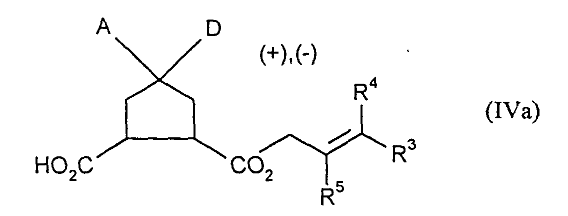 in welcher
- A, D, R³, R⁴ und R⁵: die oben angegebenen Bedeutungen haben,
überführt,
in einem weiteren Schritt im Sinn einer Curtius-Umlagerung durch Umsetzung der Verbindungen der allgemeinen Formel (IVa) mit Aziden der allgemeinen Formel (V)

(R⁶O)₂-P(O)-N₃ (V)

in welcher
- R⁶: für Phenyl oder für geradkettiges oder verzweigtes Alkyl mit bis zu 6 Kohlenstoffatomen steht,
in inerten Lösemitteln und in Anwesenheit einer Base,
oder durch Aktivierung der Carboxylgruppe der Verbindungen der allgemeinen Formel (IVa) und anschließender Reaktion mit Alkaliaziden oder Trialkylsilylaziden,
intermediär in die entsprechenden Säureazide und nachfolgend in die entsprechenden umgelagerten Isocyanaten der allgemeinen Formel (VI) überführt 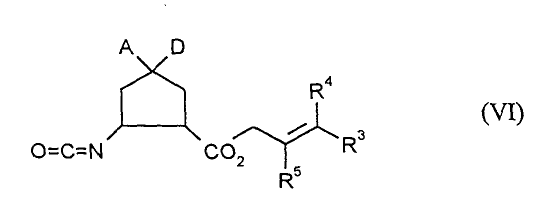 worin
- A, D, R³, R⁴ und R⁵: die vorstehend angegebenen Bedeutungen haben,
anschließend die Isocyanate mit Verbindungen der allgemeinen Formel (III) zu den Verbindungen der allgemeinen Formel (VII) 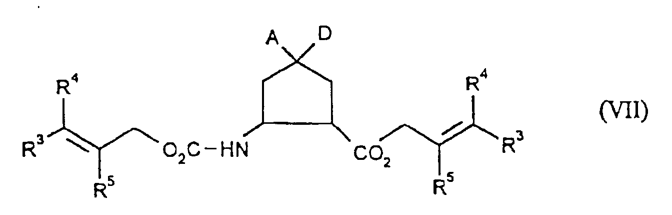 in welcher
- R³, R⁴, R⁵, A und D: die oben angegebenen Bedeutungen haben,
umsetzt
und abschließend eine Spaltung der Urethan- und der Esterfunktion in inerten Lösemitteln und in Anwesenheit eines Pd-Katalysators und/oder eines Phosphins und eines nucleophilen Hilfsstoffes durchführt,
mit der Maßgabe, daß in obiger Reaktionsfolge die Verbindungen (IV) und (VI), ohne Isolierung derselben, zur entsprechenden Folgeverbindung umgesetzt werden.

Das erfindungsgemäße Verfahren kann durch folgendes Formelschema beispielhaft erläutert werden:

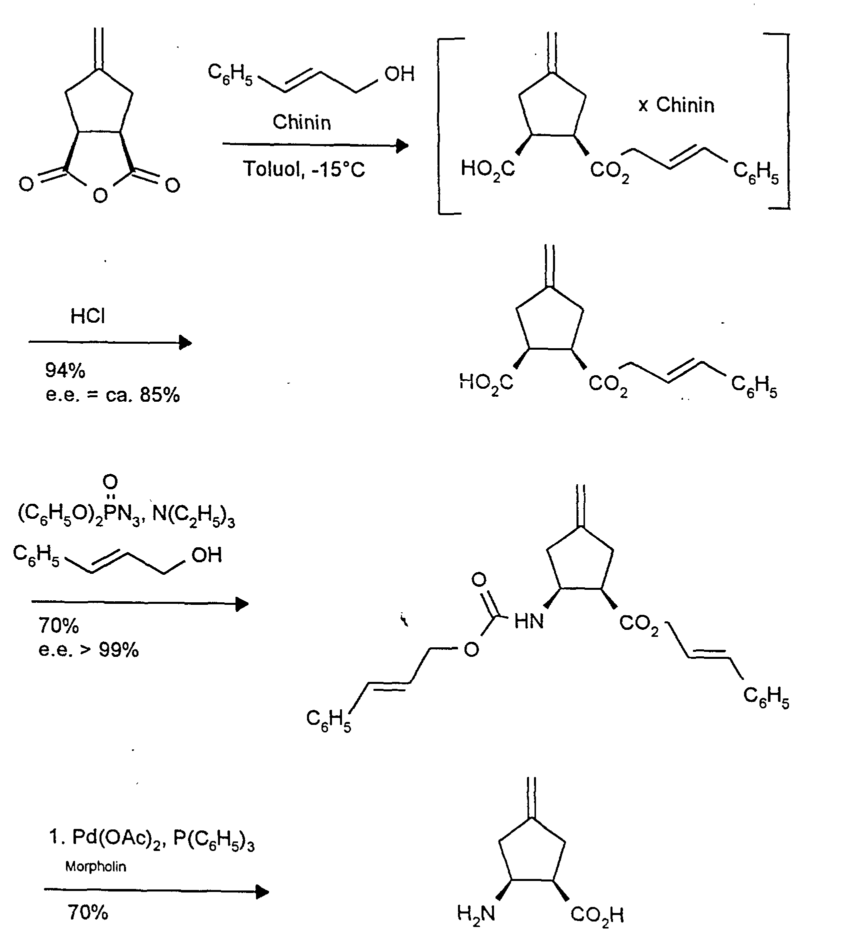

Heterocyclus im Rahmen der Erfindung steht im allgemeinen für einen aromatischen 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Furyl, Pyrrolyl, Thiazolyl, Oxazolyl oder Imidazolyl. Bevorzugt sind Pyridyl und Thienyl.

Überraschenderweise erhält man bei der Durchführung des erfindungsgemäßen Verfahrens die chiralen Verbindungen der allgemeinen Formel (I) auf elegante Weise mit sehr hoher Enantiomerenreinheit und gleichzeitig sehr guten Ausbeuten.

Im Gegensatz zum oben aufgeführten Stand der Technik ermöglicht das erfindungsgemäße Verfahren ausgehend von dem entsprechenden meso-Dicarbonsäureanhydrid über eine Curtius-Umlagerung einen hochenantioselektiven Weg zur Synthese enantiomerenreiner Cyclopentan-β-aminosäuren in einer von 6 auf 3 Stufen verkürzten Synthesesequenz mit einer Gesamtausbeute von ≥ 45% d.Th. und einem Enantiomerenüberschuß von ≥ 99%.

Ein weiterer Vorteil des erfindungsgemäßen Verfahrens liegt darin, daß die Volumenausbeute bei der Synthese der Dicarbonsäuremonoester (Formel IVa) wesentlich höher ist im Vergleich zu dem Verfahren der WO 95/19337. Darüberhinaus fällt die Zwischenisolierung der Verbindungen der allgemeinen Formel (IV) weg. Die Verbindungen der allgemeinen Formel (IVa) fallen dadurch zwar nur mit einem Enantiomerenüberschuß von 80 - >97% an, jedoch erfolgt auf der nächsten Stufe (Curtius-Umlagerung) bei der Kristallisation der Verbindungen der allgemeinen Formel (VII) eine Anreicherung auf einen Enantiomerenüberschuß von >99%.

Das erfindungsgemäße Verfahren zeichnet sich im Gegensatz zum Stand der Technik außerdem dadurch aus, daß die Hofmann-Umlagerung, Einführung und Abspaltung einer Schutzgruppe durch eine effiziente Curtius-Umlagerung ersetzt wird.

Außerdem erfolgt die Abspaltung der Urethan- und der Esterfunktion der Verbindungen der allgemeinen Formel (VII) in einem Schritt; das Produkt kristallisiert aus der Reaktionsmischung und kann in Gegensatz zum Stand der Technik durch einfaches Abfiltrieren isoliert werden.

Ein weiterer Vorteil des erfindungsgemäßen Verfahrens liegt darin, daß die Verbindungen der allgemeinen Formel (VII) im Vergleich zu den Verbindungen der Formel (V) der WO 95/19337, kristallin sind und in der Regel aus der Reaktionsmischung auskristallisieren. Dies ermöglicht eine einfachere Handhabung und durch Kristallisation eine Anreicherung der Enantiomerenreinheit zu erreichen.

Als Lösemittel für die Umsetzung der Dicarbonsäureanhydride der allgemeinen Formel (II) kommen alle inerten organischen Lösemittel in Frage, die sich unter den Reaktionsbedingungen nicht verändern. Hierzu gehören vorzugsweise Ether wie Diethylether, Dioxan, Diisopropylether, tert.Butylmethylether, Tetrahydrofuran oder Glykoldimethylether, oder Kohlenwasserstoffe wie Toluol, Benzol, Xylol, Hexan, Cyclohexan oder Erdölfraktionen, oder chlorierte Kohlenwasserstoffe, wie Chloroform oder Methylenchlorid, oder Amide wie Dimethylformamid, Dimethylacetamid oder Hexamethylphosphorsäuretriamid, oder Eisessig, Dimethylsulfoxid, Acetonitril oder Pyridin. Bevorzugt sind für die einzelnen Schritte Diisopropylether, Diethylether, Dioxan, tert.Butylmethylether und Toluol.

Die Reaktionstemperaturen können in einem größeren Bereich variiert werden. Im allgemeinen arbeitet man zwischen -60°C und +40°C, vorzugsweise zwischen -20°C und +25°C.

Die Umsetzungen können bei Normaldruck, aber auch bei erhöhtem oder erniedrigtem Druck (z.B. 0,5 bis 80 bar) durchgeführt werden. Im allgemeinen arbeitet man bei Normaldruck.

Als Alkohole für die Alkoholyse und für die Umsetzung im Sinn einer Curtius-Umlagerung (Formel III) eignen sich prim. Allylalkohole, wie beispielsweise Allyl- oder Zimtalkohol. Besonders bevorzugt ist trans-Zimtalkohol.

Als chirale Aminbasen eignen sich für das erfindungsgemäßen Verfahren bevorzugt Alkaloide und Cinchona-Alkaloide. Besonders bevorzugt sind Cinchona-Alkaloide wie beispielsweise (+), (-)-Chinin, (+), (-)-Hydrochinin, (+), (-)-Cinchonidin, (+), (-)-Epichinidin, (+), (-)-Epicinchonidin, (+), (-)-Cinchonin, (+), (-)-Epicinchonin, (+), (-)-Epichinin, (+), (-)-Hydrochinidin, (+), (-) 4-Chlorbenzoat-Epichinin oder (+), (-) 4-Chlorbenzoat-Epicinchonin. Besonders bevorzugt sind (+), (-)-Chinin und (+), (-)-Chinidin.

Die chirale Aminbase wird in equivalenten Mengen bezogen auf 1 mol der Dicarbonsäureanhyride der allgemeinen Formel (II) eingesetzt.

Als Säuren für die Rückgewinnung der freien chiralen Aminbase eignen sich beispielsweise Mineralsäuren wie Salzsäure, Bromwasserstoffsäure oder Schwefelsäure.

Die Säure wird im allgemeinen in einer Menge von 1 mol bis 10 mol, bevorzugt von 1,5 mol bis 4 mol bezogen auf 1 mol der Verbindungen der allgemeinen Formel (IV) eingesetzt.

Die Rückgewinnung erfolgt im allgemeinen in einem Temperaturbereich von 0°C bis +50°C, bevorzugt von 20°C bis 30°C und Normaldruck.

Die Curtius-Umlagerung erfolgt im allgemeinen in einem der oben aufgeführten inerten Lösemittel. Bevorzugt sind cyclische Kohlenwasserstoffe wie Benzol, Toluol oder Xylol oder Ether wie Dioxan oder Tetrahydrofuran. Bevorzugt ist Toluol.

Als Amine für die Curtius-Umlagerung eignen sich organische Amine wie N-Ethylmorpholin, N-Methylmorpholin, Pyridin, Triethylamin oder N-Methylpiperidin. Bevorzugt ist Triethylamin.

Die Base wird im allgemeinen in einer Menge von 1 mol bis 3 mol, bevorzugt von 1 mol bis 1,5 mol, bezogen auf 1 mol der Verbindungen der allgemeinen Formel (IVa) eingesetzt.

Als Azide der Formel (V) für die Curtius-Umlagerung eignen sich Phosphorsäureesterazide wie Phosphorsäurediphenylesterazid oder Phosphorsäurediethylesterazid. Bevorzugt ist Phosphorsäurediphenylesterazid.

Es ist ebenso möglich, zunächst die Carbonsäure mit Aktivierungsreagenzien wie C₁-C₄-Alkylchloroformiate in Gegenwart eines Amins, Thionylchlorid, Phosphorpentachlorid oder Phosphoroxychlorid in die entsprechenden aktivierten Derivate zu überführen und anschließend durch Umsetzung mit Alkaliaziden, wie Natriumazid oder Trialkylsilylaziden wie Trimethylsilylazid die Carbonsäureazide herzustellen.

Die Curtius-Umlagerung wird im allgemeinen in einem Temperaturbereich von 0°C bis +130°C, bevorzugt von 60°C bis 110°C, durchgeführt.

Die Curtius-Umlagerung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Aktivierung der Carboxylgruppe der Verbindungen der allgemeinen Formel (IVa) erfolgt üblicherweise mit Ethylchloroformiat/Triethylamin und im allgemeinen in einem Temperaturbereich von -30 bis +25°C.

Die derart erhaltenen Säureazide werden nachfolgend durch Erhitzen einer Lösung in einem der oben aufgeführten inerten Lösungsmittel auf Temperaturen von 60°C bis 120°C in die entsprechenden Isocyanate der allgemeinen Formel (VI) überführt.

Die Isocyanate der Formel (VI) können isoliert werden oder werden im Anschluß an ihre Herstellung mit den Alkoholen der allgemeinen Formel (III) umgesetzt.

Die Abspaltung der Urethan- und Esterfunktion in den Verbindungen der allgemeinen Formel (VII) erfolgt im allgemeinen in einem der oben aufgeführten inerten Lösemitteln. Bevorzugt sind Kohlenwasserstoffe wie Toluol, Benzol oder Xylol, Ether wie Tetrahydrofuran oder Diethylether, Ester wie Ethylacetat, Alkohole wie Ethanol, Methanol, Isopropanol, Acetonitril oder Dimethylformamid. Besonders bevorzugt sind Acetonitril, Dimethylformamid, Ethylacetat oder Ethanol.

Die Abspaltung erfolgt im allgemeinen in einem Temperaturbereich von 0°C bis +100°C, bevorzugt bei 20°C bis 80°C.

Die Abspaltung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als nucleophile Hilfsmittel für die Abspaltung eignen sich beispielsweise Carbonsäuren und deren Alkalimetallsalze (z.B. Ameisensäure, Essigsäure, 2-Ethylhexansäure, Natrium-2-ethyl-hexanoat), organische Amine wie Morpholin, Triethylamin, Pyrrolidin, Dimethyltrimethylsilylamin, Trimethylsilylmorpholin, n-Butylamin, Dimedon, Natrium-diethylmalonat, Tributylzinnhydrid, N,N-Dimethylbarbitursäure oder Ammoniumformiat. Bevorzugt ist Morpholin.

Das Hilfsmittel wird im allgemeinen in einer Menge von 1 mol bis 20 mol, bevorzugt von 1,1 mol bis 3 mol, bezogen auf 1 mol der Verbindungen der allgemeinen Formel (VII) eingesetzt.

Als Pd-Katalysatoren eignen sich im Rahmen des erfindungsgemäßen Verfahrens beispielsweise Tetrakistriphenylphosphinpalladium (O) (Pd(PPh₃)₄ Palladiumdibenzylidenaceton (Pd₂(dba)₃), Pd₂(dba)₃ x CHCl₃, Pd(dba)₂, PdCl₂, Pd(OAc)₂, PdCl₂(PhCN)₂, PdCl₂(CH₃CN)₂ oder PdCl₂(PPh₃)₂. Bevorzugt ist Palladium-II-acetat (Pd(OAc)₂).

Der Katalysator wird im allgemeinen in einer Menge von 0,0001 mol bis 0,2 mol, bevorzugt von 0,001 mol bis 0,05 mol, bezogen auf 1 mol der Verbindungen der allgemeinen Formel (VII) eingesetzt.

Als Phosphine eignen sich im allgemeinen C₁-C₄-Trialkyl- und Triarylphosphine, wie Triphenylphosphin, Triisopropylphosphin oder Tri-o-tolylphosphin. Bevorzugt ist Triphenylphosphin.

Die Verbindungen der allgemeinen Formel (II) sind an sich bekannt oder nach publizierten Methoden herstellbar.

Die Alkohole der allgemeinen Formel (III), (IIIa) und (V) sind bekannt.

Die Verbindungen der allgemeinen Formel (VII) sind neu und können wie oben beschrieben hergestellt werden.

Bevorzugt werden nach dem erfindungsgemäßen Verfahren enantiomerenreine Verbindungen der allgemeinen Formel (I) hergestellt, in welcher
- A und D: gleich oder verschieden sind und für Wasserstoff, Fluor oder für geradkettiges oder verzweigtes Alkyl mit bis zu 6 Kohlenstoffatomen stehen,
oder
- A und D: gemeinsam für einen Rest der Formel 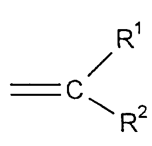 stehen,
worin
- R¹ und R²: gleich oder verschieden sind und Wasserstoff, Fluor, Brom oder geradkettiges oder verzweigtes Alkyl mit bis zu 6 Kohlenstoffatomen, Benzyl oder Phenyl bedeuten.
Besonders bevorzugt werden nach dem erfindungsgemäßen Verfahren enantiomerenreine Verbindungen der allgemeinen Formel (I) hergestellt,
in welchen
- A und D: gleich oder verschieden sind und für Wasserstoff oder für geradkettiges oder verzweigtes Alkyl mit bis zu 4 Kohlenstoffatomen stehen,
oder
- A und D: gemeinsam für einen Rest der Formel 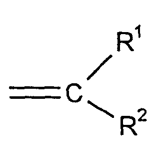 stehen,
worin
- R¹ und R²: gleich oder verschieden sind und Wasserstoff, geradkettiges oder verzweigtes Alkyl mit bis zu 4 Kohlenstoffatomen bedeuten.

Das erfindungsgemäße Verfahren ermöglicht auf effiziente, elegante und hochenantioselektive Weise und mit gleichzeitig hoher Ausbeute den Zugang zu enantiomerenreinen Cyclopentan-β-aminosäuren der allgemeinen Formel (I), die wertvolle antimykotisch und antibakteriell wirksame Arzneimittel darstellen .

### Herstellungsbeispiele

### Beispiel 1

### (1R,2S)-4-Methylencyclopentan-1,2-dicarbonsäure-1-(E)-cinnamylester

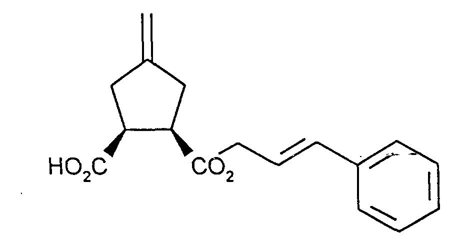

4-Methylen-1,2-cyclopentan-dicarbonsäureanhydrid (90,0 g, 591 mmol) wird in Toluol (2400 ml) unter N₂-Atmosphäre gelöst. Bei -15°C werden Chinin (191,7 g, 591 mmol) und danach trans-Zimtalkohol (119,2 g, 888 mmol) zugegeben. Die Reaktionsmischung wird mind. 4 h bei -15°C gerührt. Man läßt auf Raumtemperatur erwärmen und wäscht mit 1 N HCl (3 x 900 ml) und Wasser (2 x 900 ml). Das Produkt wird anschließend aus der organischen Phase mit 2%iger wäßriger K₂CO₃-Lösung (1 x 4,5 l, 2 x 1,5 l) extrahiert. Die vereinigten, wäßrigen Phasen werden mit Ethylacetat gewaschen (2 x 1 l), mit Toluol (600 ml) überschichtet und unter starkem Rühren mit 10%iger Salzsäure auf pH 2 gestellt. Nach Phasentrennung wird noch zweimal mit Toluol extrahiert (2 x 600 ml). Die vereinigten Toluolphasen werden mit Wasser (2 x 400 ml) gewaschen und im Vakuum bei 50°C / ca. 20 mbar eingeengt.
Ausbeute: 159,1 g, 94% d.Th.
Enantiomerenüberschuß e.e. ≥ 85% (HPLC, Chiracel OD-H, Eluent: n-Heptan/Isopropanol).
Wird das Produkt anschließend mit Toluol (180 ml) versetzt und die entstandene Suspension ca. 1 h gerührt, so erhält man nach Filtration und Eindampfen des Filtrates im Vakuum (1R,2S)-4-Methylencyclopentan-1,2-dicarbonsäure-1-(E)-cinnamylester (144,6 g, 85% d.Th.) mit einem Enantiomerenüberschuß von e.e. ≥ 98% (HPLC, Chiracel OD-H, Eluent: n-Heptan / Isopropanol).

| C₁₇H₁₈O₄ (286.3) | | |
|---|---|---|
| theor. | C 71,31% | H 6,34% |
| gef. | C 71,27% | H 6,42% |

### Beispiel 1a

### (1S,2R)-4-Methylencyclopentan-1,2-dicarbonsäure-1-(E)-cinnamylester

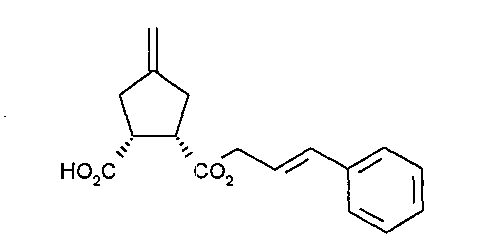

Eine Suspension von Chinidin (179,4 g, 553 mmol) wird unter N₂-Atmosphäre auf -15°C gekühlt und nacheinander mit 4-Methylen-cyclopentan-1,2-dicarbonsäureanhydrid (84,0 g, 553 mmol) und trans-Zimtalkohol (111,2 g, 829 mmol) versetzt. Die Reaktionsmischung wird mindestens 4 h bei -15°C gerührt. Die Aufarbeitung wird in Analogie zur Herstellung der Verbindung aus Beispiel 1 durchgeführt.
Ausbeute: 147,3 g, 93% d.Th.
Enantiomerenüberschuß e.e. ≥ 93% (HPLC, Chiracel OD-H, Eluent: n-Heptan/Isopropanol).

| C₁₇H₁₈O₄ (286.3) | | |
|---|---|---|
| theor. | C 71,31% | H 6,34% |
| gef. | C 71,23% | H 6,32% |

### Beispiel 2

### (1R,2S)-2-N-((E)-Cinnamyloxycarbonyl)amino-4-methylen-1-cyclopentan-carbonsäure-(E)-cinnamylester

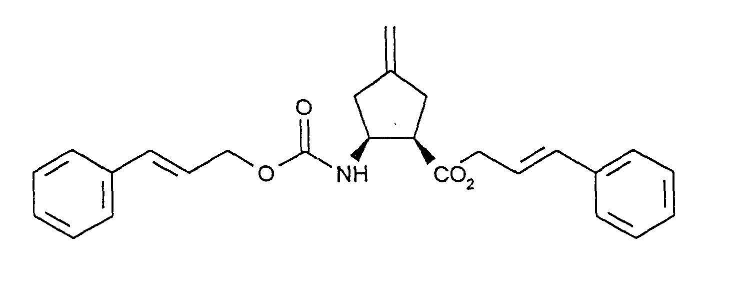

Zur Lösung der Verbindung aus Beispiel 1 (10,0 g, 34,9 mmol, e.e. ≥ 85%) in Toluol (70 ml) tropft man unter Stickstoffatmosphäre nacheinander Triethylamin (3,5 g, 34,7 mmol) und Phosphorsäurediphenylesterazid (9,6 g, 34,9 mmol).
Man erhitzt die Reaktionsmischung ca. 30 min auf 90°C, bis kein Stickstoff mehr entweicht. Anschließend tropft man bei 90°C trans-Zimtalkohol (5,6 g, 41,9 mmol) zu und erhitzt über Nacht unter Stickstoff zum Rückfluß. Man läßt den Ansatz unter Rühren auf Raumtemperatur abkühlen, kühlt mit einem Eisbad weiter bis auf ca. 3°C, saugt ausgefallenes Produkt ab, wäscht mit insgesamt 50 ml kaltem Toluol und trocknet das Produkt im Vakuum bei 50°C.
Ausbeute: 10,3 g, 70% d.Th., weiße Kristalle
Enantiomerenüberschuß e.e. ≥ 99% (HPLC, Chiracel OD-H, Eluent: n-Heptan / Isopropanol + Trifluoressigsäure).
Smp.: 136°C

| C₂₆H₂₇NO₄ (417,51) | | | |
|---|---|---|---|
| theor. | C 74,80% | H 6,52% | N 3,36% |
| gef. | C 74,88% | H 6,44% | N 3,51% |

Führt man die oben beschriebene Reaktion mit der Verbindung aus Beispiel 1 mit einem Enantiomerenüberschuß von e.e. ≥ 98 % unter ansonsten analogen Bedingungen durch, so erhält man das Produkt in einer Ausbeute von 11,7 g (80% d.Th.) mit einem Enantiomerenüberschuß von ≥ 99%.

### Beispiel 2a

### (1S,2R)-2-N-((E)-Cinnamyloxycarbonyl)amino-4-methylen-1-cyclopentan-carbonsäure-(E)-cinnamylester

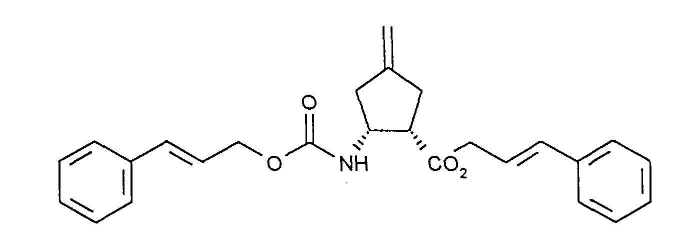

Die Herstellung gelingt in Analogie zur Herstellung der Verbindung aus Beispiel 2 ausgehend von der Verbindung aus Beispiel 1a (30,0 g, 104,8 mmol).
Ausbeute: 34,6 g, 79,1 % d.Th.,
Enantiomerenüberschuß e.e. ≥ 99% (HPLC, Chiracel OD-H, Eluent: n-Heptan / Isopropanol + Trifluoressigsäure).
Smp.: 137°C

| C₂₆H₂₇NO₄ (417,51) | | | |
|---|---|---|---|
| theor. | C 74,80% | H 6,52% | N 3,36% |
| gef. | C 74,99% | H 6,63% | N 3,34% |

### Beispiel 3

### (1R,2S)-2-N-((E)-Cinnamyloxycarbonyl)-amino-4-methylen-1-cyclopentancarbonsäure-allylester

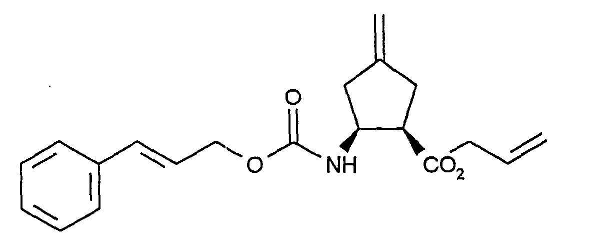

Die Herstellung erfolgt analog wie für Beispiel 2 beschrieben, ausgehend von (1R,2S)-4-Methylen-cyclopentan-1,2-dicarbonsäure-1-allylester (DE 44 007 49 A1; 7,3 g, 34,9 mmol, e.e. ≥ 96%).
Ausbeute: 7,9 g, 66% d.Th., weiße Kristalle

| C₂₀H₂₃NO₄ (341,38) | | | |
|---|---|---|---|
| theor. | C 70,36% | H 6,79% | N 4,10% |
| gef. | C 70,25% | H 6,97% | N 4,08% |

### Beispiel 4

### (-)-(1R,2S)-2-Amino-4-methylen-cyclopentan-1-carbonsäure

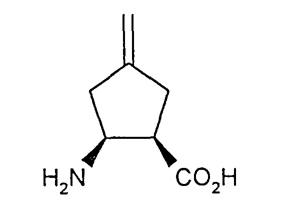

Eine Lösung der Verbindung aus Beispiel 2 (180,0 g, 431 mmol) in Ethylacetat (1500 ml) wird unter N₂-Atmosphäre nacheinander versetzt mit Triphenylphosphin (5,38 g, 20,5 mmol), Morpholin (75,1 g, 862 mmol) und Palladium-(II)-acetat (0,97 g, 4,3 mmol). Die Reaktionsmischung wird 2 h zum Rückfluß erhitzt und danach auf ca. 60°C abgekühlt. Ausgefallenes Produkt wird abgesaugt, mit Ethylacetat gewaschen und i.V. getrocknet. Das Rohprodukt wird zweimal aus 85%igem wäßrigem Ethanol umkristallisiert.
Ausbeute: 42,6 g, 70% d.Th., weiße Kristalle
Smp.: 222°C
[α]²⁰_{D} = -31.6 (c=1, H₂O)

| C₇H₁₁NO₂ (141,2) | | | |
|---|---|---|---|
| theor. | C 59,56% | H 7,85% | N 9,92% |
| gef. | C 59,46% | H 7,85% | N 9,88% |

Analog und in nahezu gleicher Ausbeute gelingt die Herstellung der Verbindung aus Beispiel 4 ausgehend von der Verbindung aus Beispiel 3.

### Beispiel 4a

### (+)-(1S,2R)-2-Amino-4-methylen-cyclopentan-1-carbonsäure

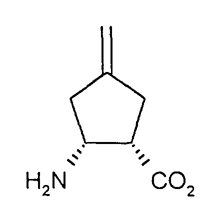

Die Herstellung gelingt in Analogie zur Herstellung der Verbindung aus Beispiel 4 ausgehend von der Verbindung aus Beispiel 2a (60,3 g, 144,4 mmol).

Ausbeute: 12,4 g, 67,6 % d.Th.

Smp.: 233°C (Zers.)
[α]²⁰_{D} = +32,2 (c =1,02, H₂O)

| | | | |
|---|---|---|---|
| theor. | C 59,56% | H 7,85% | N 9,92% |
| gef. | C 59,09% | H 7,74% | N 9,87% |

### Beispiel 5

### (1R, 2S)-Cyclopentan-1,2-dicarbonsäure-1-(E)-cinnamylester

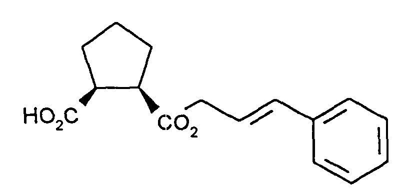

Eine Suspension von Chinin (50,9 g, 157,1 mmol) in Toluol (634 ml) wird unter N₂-Atmosphäre bei -15°C nacheinander mit Cyclopentan-1,2-dicarbonsäureanhydrid (22,0 g, 157,1 mmol) und trans-Zimtalkohol (31,6 g, 235,7 mmol) versetzt. Die Reaktionsmischung wird mindestens 4 h bei -15°C gerührt. Man läßt auf Raumtemperatur erwärmen und wäscht mit IN HCl (2x 240 ml) und Wasser (240 ml). Das Produkt wird anschließend mit 2,2 %iger, wäßrige K₂CO₃-Lösung (1 x 1210 ml, 1 x 400 ml) aus der organischen Phase extrahiert. Die vereinigten, wäßrigen Phasen werden mit Ethylacetat (2 x 260 ml) gewaschen, mit Toluol (260 ml) überschichtet und unter starkem Rühren mit 10 %ige Salzsäure auf pH 2 gestellt. Nach Phasentrennung wird noch einmal mit Toluol (260 ml) extrahiert. Die vereinigten Toluolphasen werden mit Waser (2 x 130 ml) gewaschen und im Vakuum bei 50°C eingeengt.
Ausbeute: 40,2 g (93 % d.Th.)
Enantiomerenüberschuß e.e. ≥ 86% (HPLC)

| C₁₆H₁₈O₄ (274,3) | | |
|---|---|---|
| theor. | C 70,06% | H 6,61% |
| gef. | C 69,66% | H 6,42% |

### Beispiel 6

### (1R,2S)-2N-((E)-Cinnamyloxycarbonyl)amino-cyclopentan-1-carbonsäure-(E)-cinnamylester

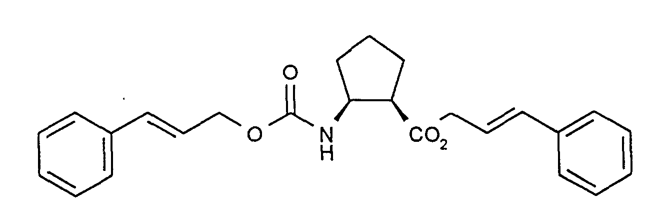

Zur Lösung der Verbindung aus Beispiel 5 (39,3 g, 143 mmol) in Toluol (286 ml) tropft man unter N₂-Atmosphäre nacheinander Triethylamin (14,3 g, 142 mmol) und Phosphorsäurediphenylesterazid (39,4 g, 143 mmol).
Man erhitzt die Reaktionsmischung 30 min auf 90°C und tropft anschließend bei dieser Temperatur trans-Zimtalkohol (23,0 g, 172 mmol) zu und erhitzt über Nacht zum Rückfluß. Man läßt den Ansatz unter Rühren auf 30°C abkühlen, saugt ausgefallenes Rohprodukt (14,2 g) ab und wäscht mit kaltem Toluol (60 ml) nach.
Die Mutterlauge wird nacheinander gewaschen mit 5 %iger, wäßriger Zitronensäurelösung (410 ml), Wasser (410 ml), ges. NaHCO₃-Lösung (410 ml) und ges. NaCl-Lösung (410 ml). Nach dem Einengen der organischen Phase im Vakuum wird weiteres Rohprodukt (30,0 g) erhalten.
Das Rohprodukt wird anschließend aus Isopropanol umkristallisiert.
Ausbeute: 33,3 g (57,3 % d.Th.), weiße Kristalle
Enantiomerenüberschuß e.e. ≥ 99% (HPLC, Chiracel OD-H)
Smp.: 84-85°C

| C₂₅H₂₇NO₄ (405,5) | | | |
|---|---|---|---|
| theor. | C 74,05% | H 6,71% | N 3,45% |
| gef. | C 74,12% | H 6,58% | N 3,53% |

### Beispiel 7

### (-)-(1R, 2S)-2-Amino-cyclopentan-1-carbonsäure

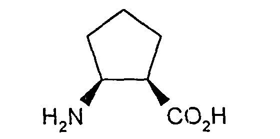

Eine Lösung der Verbindung aus Beispiel 6 (44,8 g, 110,5 mmol) und Triphenylphosphin (1,37 g, 5,2 mmol) in Ethanol (127 ml) wird unter N₂-Atmosphäre mit Morpholin (19,3 g, 221 mmol) und Palladium-(II)-acetat (0,062 g, 0,28 mmol) versetzt. Die Reaktionsmischung wird 2 h zum Rückfluß erhitzt, mit 3-Mercapto-1,2,4-triazol (1,12 g, 11,1 mmol) versetzt, weitere 1,5 h unter Rückfluß erhitzt und danach auf 0-5°C abgekühlt. Ausgefallenes Rohprodukt wird abgesaugt, mit Ethanol gewaschen und im Vakuum getrocknet. Das Rohprodukt wird aus 85 %igem, wäßrigen Ethanol in Gegenwart von 5 Mol-% Mercapto-1,2,4-triazol umkristallisiert.
Ausbeute: 9,4 g (66 % d.Th.), weiße Kristalle
Smp.: 218°C (Zers.)
[α]²⁰_{D} = 9,9 (c =1,0, H₂O)

| C₆H₁₁NO₂ (129,2) | | | |
|---|---|---|---|
| theor. | C 55,80% | H 8,59% | N 10,84% |
| gef. | C 55,53% | H 8,24% | N 10,83% |

## Patentansprüche

1. Verfahren zur Herstellung von enantiomerenreinen Cyclopentan-β-aminosäuren der allgemeinen Formel (I) 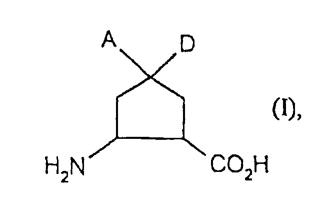 in welcher
A und D gleich oder verschieden sind und für Wasserstoff, Halogen, Hydroxy, oder für geradkettiges oder verzweigtes Alkyl mit bis zu 8 Kohlenstoffatomen, das gegebenenfalls 1- bis 2-fach gleich oder verschieden durch Halogen, Hydroxy, Phenyl, Benzyloxy, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR⁷R⁸ substituiert ist,
worin R⁷ und R⁸ gleich oder verschieden sind und Wasserstoff, Phenyl oder geradkettiges oder verzweigtes Alkyl mit bis zu 6 Kohlenstoffatomen bedeuten, stehen
oder
A und D gemeinsam für einen Rest der Formel 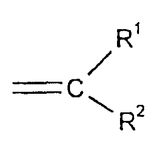 stehen,
worin
R¹ und R² gleich oder verschieden sind und Wasserstoff, Halogen oder geradkettiges oder verzweigtes Alkyl, Alkoxy oder Oxyacyl mit bis zu 8 Kohlenstoffatomen, Benzyl oder Phenyl bedeuten,
**dadurch gekennzeichnet, daß** man meso-Dicarbonsäureanhydride der allgemeinen Formel (II) 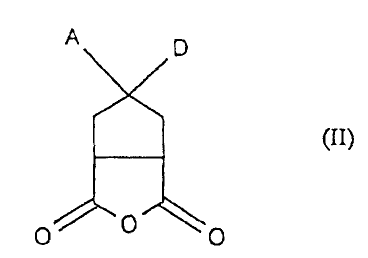 in welcher
A und D die oben angegebenen Bedeutungen haben,
durch eine asymmetrische Alkoholyse mit Allylalkoholen der allgemeinen Formel (III) 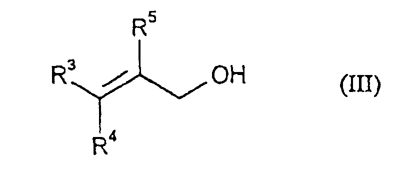 in welcher
R³, R⁴ und R⁵ gleich oder verschieden sind und für Wasserstoff oder für geradkettiges oder verzweigtes Alkyl mit bis zu 5 Kohlenstoffatomen oder für Phenyl stehen, das gegebenenfalls bis zu 3-fach gleich oder verschieden durch Halogen, Cyano, Trifluormethoxy, Nitro, Trifluormethyl oder durch geradkettiges oder verzweigtes Alkyl oder Alkoxy mit jeweils bis zu 6 Kohlenstoffatomen substituiert ist,
oder
R³ für einen 5- bis 7-gliedrigen aromatischen Heterocyclus mit bis zu 3 Heteroatomen aus der Reihe S, N und/oder O steht,
und in Anwesenheit equimolarer Mengen einer in enantiomerenreiner Form vorliegenden chiralen Aminbase, in inerten Lösemitteln, zunächst über die intermediäre, enantiomerenreine Salzstufe der allgemeinen Formel (IV) 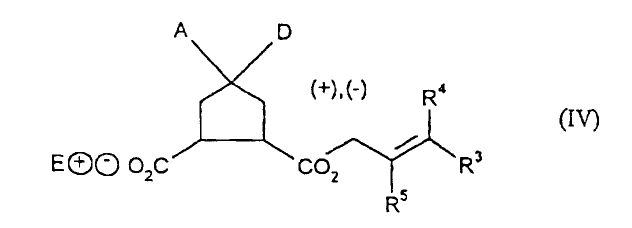 in welcher
A, D, R³, R⁴ und R⁵ die oben angegebenen Bedeutungen haben
und
E für die chirale Aminbase steht,
in die enantiomerenreinen Verbindungen der allgemeinen Formel (IVa) 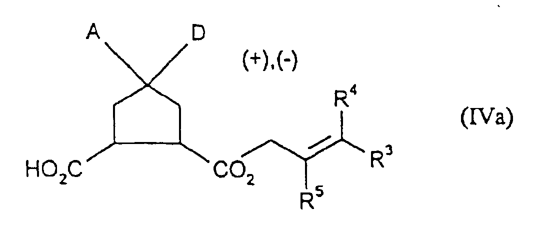 in welcher
A, D, R³, R⁴ und R⁵ die oben angegebenen Bedeutungen haben,
überführt,
in einem weiteren Schritt im Sinn einer Curtius-Umlagerung durch Umsetzung der Verbindungen der allgemeinen Formel (IVa) mit Aziden der allgemeinen Formel (V)
(R⁶O)₂-P(O)-N₃ (V)
in welcher
R⁶ für Phenyl oder für geradkettiges oder verzweigtes Alkyl mit bis zu 6 Kohlenstoffatomen steht,
in inerten Lösemitteln und in Anwesenheit einer Base,
oder durch Aktivierung der Carboxylgruppe der Verbindungen der allgemeinen Formel (IVa) und anschließender Reaktion mit Alkaliaziden oder Trialkylsilylaziden,
intermediär in die entsprechenden Säureazide und nachfolgend in die entsprechenden umgelagerten Isocyanate der allgemeinen Formel (VI) überführt 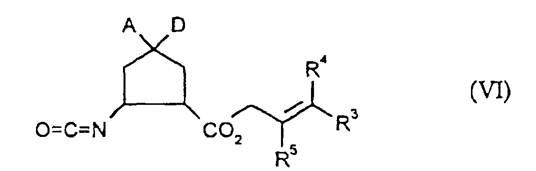 worin
A, D, R³, R⁴ und R⁵ die vorstehend angegebenen Bedeutungen haben,
anschließend die Isocyanate mit Verbindungen der allgemeinen Formel (III) zu den Verbindungen der allgemeinen Formel (VII) 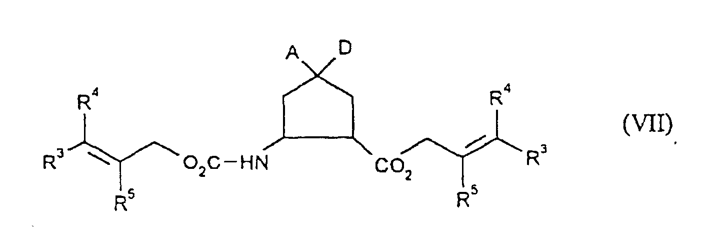 in welcher
R³, R⁴, R⁵, A und D die oben angegebenen Bedeutungen haben,
umsetzt
und abschließend eine Spaltung der Urethan- und der Esterfunktion in inerten Lösemitteln und in Anwesenheit eines Pd-Katalysators und/oder eines Phosphins und eines nucleophilen Hilfsstoffes durchführt,
mit der Maßgabe, daß in obiger Reaktionsfolge die Verbindungen (IV) und (VI), ohne Isolierung derselben, zur entsprechenden Folgeverbindung umgesetzt werden.

2. Verfahren nach Anspruch 1, **dadurch gekennzeichnet, daß** die Umsetzung des Dicarbonsäureanhydrids der allgemeinen Formel (II) in einem Temperaturbereich zwischen -60°C und +40°C, vorzugsweise zwischen -20°C und +25°C durchgeführt wird.

3. Verfahren nach Anspruch 1, **dadurch gekennzeichnet, daß** die Umsetzung des Dicarbonsäureanhydrids der allgemeinen Formel (II) bei einem Druck im Bereich von 0,5 bis 80 bar, bevorzugt Normaldruck erfolgt.

4. Verfahren nach Anspruch 1, **dadurch gekennzeichnet, daß** die Curtius-Umlagerung in einem Temperaturbereich zwischen 0 bis +130°C, bevorzugt 6 bis 110°C durchgeführt wird.

5. Verfahren nach Anspruch 1, **dadurch gekennzeichnet, daß** die Curtius-Umlagerung bei einem Druck im Bereich von 0,5 bis 5 bar, bevorzugt Normaldruck durchgeführt wird.

6. Verfahren nach Anspruch 1, **dadurch gekennzeichnet, daß** bei der Curtius-Umlagerung die Base in einer Menge von 1 bis 3 mol, bevorzugt 1 bis 1,5 mol, bezogen auf 1 mol der Verbindung der allgemeinen Formel (IVa) eingesetzt wird.

7. Verfahren nach Anspruch 1, **dadurch gekennzeichnet, daß** die Abspaltung der Urethan- und Esterfunktion in einem Temperaturbereich von 0°C bis 100°C, bevorzugt bei 20°C bis 80°C durchgeführt wird.

8. Verfahren nach Anspruch 1, **dadurch gekennzeichnet, daß** die Abspaltung der Urethan- und Esterfunktion bei einem Druck im Bereich von 0,5 bis 5 bar, bevorzugt bei Normaldruck erfolgt.

9. Enantiomerenreine Verbindungen der allgemeinen Formel (VII) 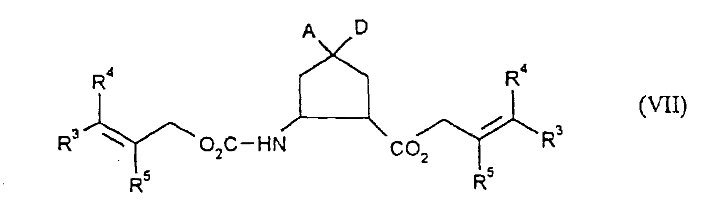 in welchen
R³, R⁴, R⁵, A und D die in Anspruch 1 angegebenen Bedeutungen haben.

## Claims

1. Process for the preparation of enantiomerically pure cyclopentane-β-amino acids of the general formula (I) 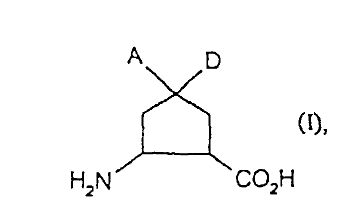 in which
A and D are identical or different and represent hydrogen, halogen or hydroxyl, or represent straight-chain or branched alkyl having up to 8 carbon atoms, which is optionally substituted once or twice in an identical or different manner by halogen, hydroxyl, phenyl, benzyloxy or carboxyl or by straight-chain or branched alkoxy, acyl or alkoxycarbonyl having in each case up to 6 carbon atoms or by a group of the formula -NR⁷R⁸,
wherein R⁷ and R⁸ are identical or different and denote hydrogen, phenyl or straight-chain or branched alkyl having up to 6 carbon atoms,
or
A and D together represent a radical of the formula 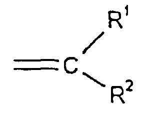 wherein
R¹ and R² are identical or different and denote hydrogen, halogen or straight-chain or branched alkyl, alkoxy or hydroxyacyl having up to 8 carbon atoms, benzyl or phenyl,
**characterized in that** meso-dicarboxylic acid anhydrides of the general formula (II) 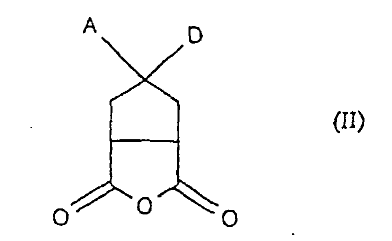 in which
A and D have the abovementioned meanings,
are first converted by an asymmetric alcoholysis with allyl alcohols of the general formula (III) 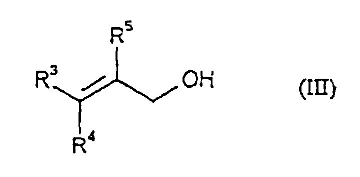 in which
R³, R⁴ and R⁵ are identical or different and represent hydrogen, or represent straight-chain or branched alkyl having up to 5 carbon atoms, or represent phenyl, which is optionally substituted up to 3 times in an identical or different manner by halogen, cyano, trifluoromethoxy, nitro or trifluoromethyl or by straight-chain or branched alkyl or alkoxy having in each case up to 6 carbon atoms,
or
R³ represents a 5- to 7-membered aromatic heterocyclic radical having up to 3 heteroatoms from the series consisting of S, N and/or O,
and in the presence of equimolar amounts of a chiral amine base present in enantiomerically pure form, in inert solvents, via the intermediate enantiomerically pure salt stage of the general formula (IV) 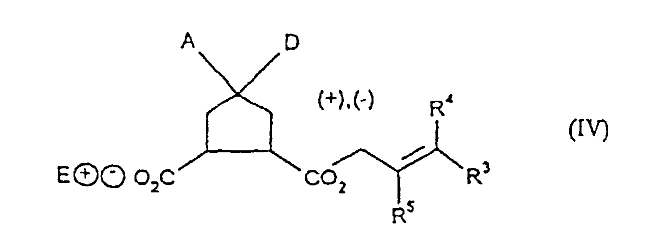 in which
A, D, R³, R⁴ and R⁵ have the abovementioned meanings
and
E represents the chiral amine base,
into the enantiomerically pure compounds of the general formula (IVa) 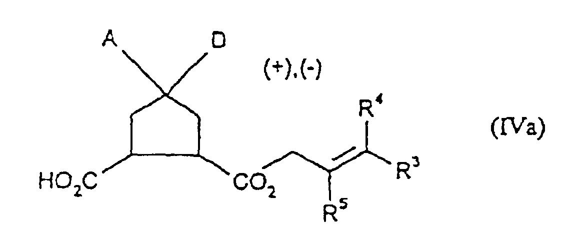 in which
A, D, R³, R⁴ and R⁵ have the abovementioned meanings,
in a further step are intermediately converted, in the sense of a Curtius rearrangement by reaction of the compounds of the general formula (IVa) with azides of the general formula (V)
(R⁶O)₂-P(O)-N₃ (V)
in which
R⁶ represents phenyl, or represents straight-chain or branched alkyl having up to 6 carbon atoms,
in inert solvents and in the presence of a base, or by activation of the carboxyl group of the compounds of the general formula (IVa) and subsequent reaction with alkali metal azides or trialkylsilyl azides,
into the corresponding acid azides, and are subsequently converted into the corresponding rearranged isocyanates of the general formula (VI) 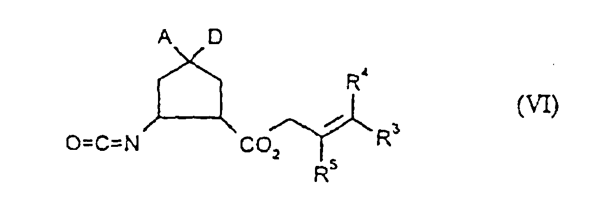 wherein
A, D, R³, R⁴ and R⁵ have the abovementioned meanings,
and the isocyanates are then reacted with compounds of the general formula (III) to give the compounds of the general formula (VII) 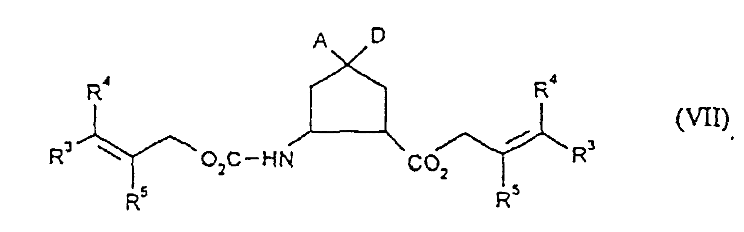 in which
R³, R⁴, R⁵, A and D have the abovementioned meanings,
and finally the urethane and the ester function are split in inert solvents and in the presence of a Pd catalyst and/or a phosphine and a nucleophilic auxiliary,
with the proviso that in the above reaction sequence, the compounds (IV) and (VI), without being isolated, are reacted to give the corresponding sequence compound.

2. Process according to Claim 1, **characterized in that** the reaction of the dicarboxylic acid anhydride of the general formula (II) is carried out in a temperature range between -60°C and +40°C, preferably between -20°C and +25°C.

3. Process according to Claim 1, **characterized in that** the reaction of the dicarboxylic acid anhydride of the general formula (II) is carried out under a pressure in the range from 0.5 to 80 bar, preferably normal pressure.

4. Process according to Claim 1, **characterized in that** the Curtius rearrangement is carried out in a temperature range between 0 and +130°C, preferably 6 and 110°C.

5. Process according to Claim 1, **characterized in that** the Curtius rearrangement is carried out under a pressure in the range from 0.5 to 5 bar, preferably normal pressure.

6. Process according to Claim 1, **characterized in that**, in the Curtius rearrangement, the base is employed in an amount of 1 to 3 mol, preferably 1 to 1.5 mol, per mole of the compound of the general formula (IVa).

7. Process according to Claim 1, **characterized in that** the urethane and ester function are split off in a temperature range from 0°C to 100°C, preferably at 20°C to 80°C.

8. Process according to Claim 1, **characterized in that** the urethane and ester function are split off under a pressure in the range from 0.5 to 5 bar, preferably under normal pressure.

9. Enantiomerically pure compounds of the general formula (VII) 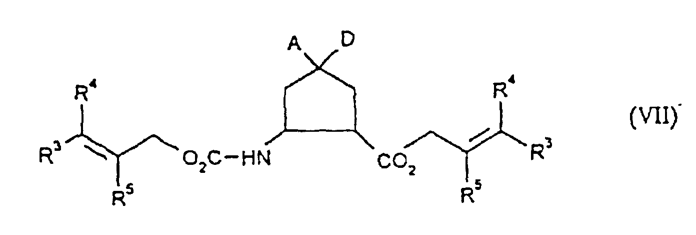 in which
R³, R⁴, R⁵, A and D have the meanings given in Claim 1.

## Revendications

1. Procédé de production de cyclopentane-β-amino-acides énantiomériquement purs de formule générale (I) 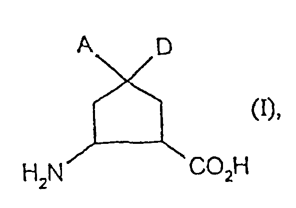 dans laquelle
A et D sont identiques ou différents et représentent l'hydrogène, un halogène, un groupe hydroxy ou bien un groupe alkyle linéaire ou ramifié ayant jusqu'à 8 atomes de carbone, qui est substitué le cas échéant une ou deux fois identiques ou différentes par un radical halogéno, hydroxy, phényle, benzyloxy, carboxy ou par un radical alkoxy, acyle ou alkoxycarbonyle linéaire ou ramifié ayant dans chaque cas jusqu'à 6 atomes de carbone ou par un groupe de formule -NR⁷R⁸, où R⁷ et R⁸ sont identiques ou différents et représentent l'hydrogène, un groupe phényle ou un groupe alkyle linéaire ou ramifié ayant jusqu'à 6 atomes de carbone,
ou bien
A et D forment ensemble un reste de formule 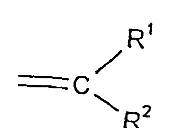 où
R¹ et R² sont identiques ou différents et représentent l'hydrogène, un halogène ou un groupe alkyle, alkoxy ou oxy-acyle linéaire ou ramifié ayant jusqu'à 8 atomes de carbone, un groupe benzyle ou phényle,
**caractérisé en ce qu'**on transforme tout d'abord des anhydrides d'acides mésodicarboxyliques de formule générale (II) 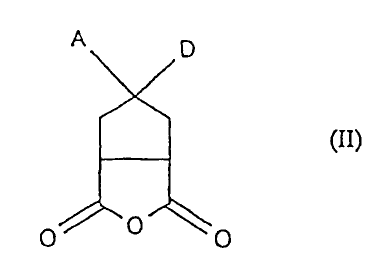 dans laquelle
A et D ont les définitions indiquées ci-dessus,
par une alcoolyse asymétrique avec des alcools allyliques de formule générale (III) 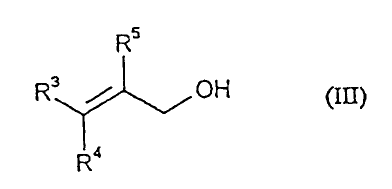 dans laquelle
R³, R⁴ et R⁵ sont identiques ou différents et représentent l'hydrogène ou un groupe alkyle linéaire ou ramifié ayant jusqu'à 5 atomes de carbone ou le groupe phényle, qui est substitué le cas échéant jusqu'à 3 fois identiques ou différentes par un radical halogéno, cyano, trifluorométhoxy, nitro, trifluorométhyle ou par un radical alkyle ou alkoxy linéaire ou ramifié ayant chacun jusqu'à 6 atomes de carbone,
ou bien
R³ est un hétérocycle aromatique pentagonal à heptagonal ayant jusqu'à 3 hétéro-atomes de la série S, N et/ou O,
et en présence de quantités équimolaires d'une base aminée chirale présente sous une forme énantiomériquement pure, dans des solvants inertes, en passant par le stade intermédiaire de sel énantiomériquement pur de formule générale (IV) 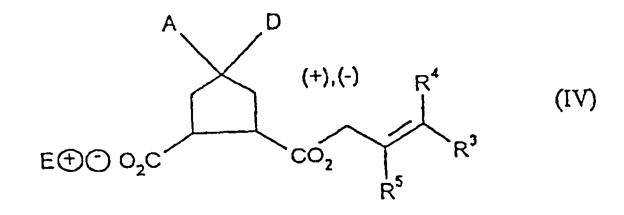 dans laquelle
A, D, R³, R⁴ et R⁵ ont les définitions indiquées ci-dessus
et
E représente une base aminée chirale,
en les composés énantiomériquement purs de formule générale (IVa) 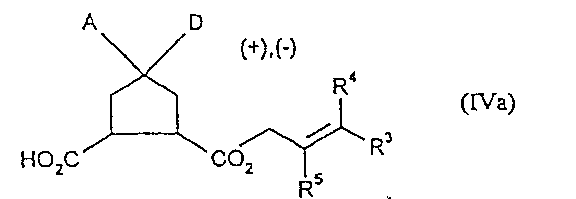 dans laquelle
A, D, R³, R⁴ et R⁵ ont les définitions indiquées ci-dessus,
on conduit une transformation intermédiaire dans une autre étape, au sens d'une transposition de Curtius, par réaction des composés de formule générale (IVa) avec des azides de formule générale (V)
(R⁶O)₂-P(O) -N₃ (V)
dans laquelle
R⁶ est un groupe phényle ou un groupe alkyle linéaire ou ramifié ayant jusqu'à 6 atomes de carbone,
dans des solvants inertes et en présence d'une base,
ou par activation du groupe carboxyle des composés de formule générale (IVa) suivie d'une réaction avec des azotures alcalins ou des trialkylsilylazides,
en les azides d'acides correspondants puis en les isocyanates transposés correspondants de formule générale (VI) 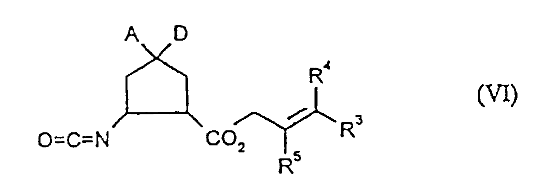 dans laquelle
A, D, R³, R⁴ et R⁵ ont les définitions indiquées ci-dessus,
après quoi on fait réagir les isocyanates avec des composés de formule générale (III) pour obtenir les composés de formule générale (VII) 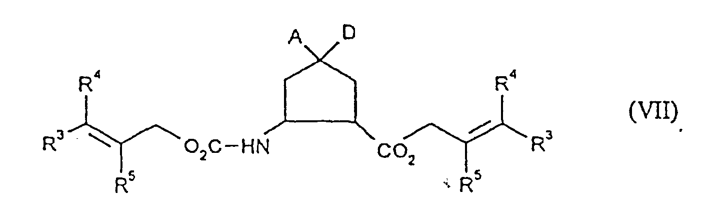 dans laquelle
R³, R⁴, R⁵, A et D ont les définitions indiquées ci-dessus,
et finalement on conduit une élimination de la fonction uréthanne et de la fonction ester dans des solvants inertes et en présence d'un catalyseur au palladium et/ou d'une phosphine et d'une substance auxiliaire nucléophile,
étant entendu que dans les réactions successives indiquées ci-dessus, les composés (IV) et (VI) réagissent sans qu'on les isole pour former le dérivé correspondant.

2. Procédé suivant la revendication 1,
**caractérisé en ce que** la réaction de l'anhydride d'acide dicarboxylique de formule générale (II) est conduite dans une plage de températures entre -60°C et +40°C, de préférence entre -20°C et +25°C.

3. Procédé suivant la revendication 1,
**caractérisé en ce que** la réaction de l'anhydride d'acide dicarboxylique de formule générale (II) s'accomplit à une pression comprise dans la plage de 0,5 à 80 bars, de préférence sous pression normale.

4. Procédé suivant la revendication 1,
**caractérisé en ce que** la transposition de Curtius est conduite dans une plage de températures entre 0 et +130°C, de préférence entre 6 et 110°C.

5. Procédé suivant la revendication 1,
**caractérisé en ce que** la transposition de Curtius est conduite à une pression comprise dans la plage de 0,5 à 5 bars, de préférence sous pression normale.

6. Procédé suivant la revendication 1,
**caractérisé en ce que** la base est utilisée dans la transposition de Curtius en une proportion de 1 à 3 moles, de préférence de 1 à 1,5 mole par mole du composé de formule générale (IVa).

7. Procédé suivant la revendication 1,
**caractérisé en ce que** l'élimination de la fonction uréthanne et de la fonction ester est conduite dans une plage de températures de 0°C à 100°C, de préférence de 20°C à 80°C.

8. Procédé suivant la revendication 1,
**caractérisé en ce que** l'élimination de la fonction uréthanne et de la fonction ester est conduite à une pression comprise dans la plage de 0,5 à 5 bars, de préférence sous pression normale.

9. Composés énantiomériquement purs de formule générale (VII) 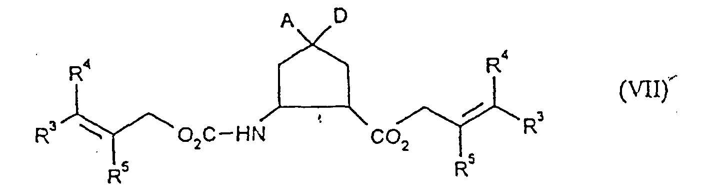 dans lesquels
R³, R⁴, R⁵, A et D ont les définitions indiquées dans la revendication 1.
